# FASCICULE DE BREVET EUROPEEN

(11) **EP 2 287 275 B1**
(45) Date de publication et mention de la délivrance du brevet: **06.03.2013**
(21) Numéro de dépôt: 10290403.4
(22) Date de dépôt: 16.07.2010
(51) Int. Cl.: C10G 45/36, B01J 23/755, B01J 23/89, B01J 37/02, C07C 5/02

(54) **Procédé de préparation d'un catalyseur supporte à base de Ni et d'un metal du groupe IB pour l'hydrogénation sélective d'hydrocarbures polyinsaturés**
Verfahren zur Herstellung eines geträgerten Katalysators auf Basis von Ni und eines Metalls der Gruppe IB zur selektiven Hydrierung polyungesättigter Kohlenwasserstoffe
Process for preparing a supported catalyst based on Ni and a Group IB metal for selective hydrogenation of polyunsaturated hydrocarbons

(30) Priorité: 17.08.2009 FR 0903987
(43) Date de publication de la demande: 23.02.2011
(73) Titulaire: IFP Energies nouvelles, 92852 Rueil-Malmaison Cedex (FR)
(72) Inventeur: Fischer, Lars, 38200 Veinne (FR); Dubreuil, Anne-Claire, 69003 Lyon (FR); Thomazeau, Cecile, 69008 Lyon (FR); Deghedi, Layane, 69007 Lyon (FR); Candy, Jean-Pierre, 69300 Caluire et Cuire (FR); Basset, Jean-Marie, 69300 Caluire et Cuire (FR); Le Peltier, Fabienne, 92500 Rueil Malmaison (FR)

(56) Documents cités:
- WO-A1-2006/009988
- FR-A1- 2 764 210
- FR-A1- 2 764 299
- FR-A1- 2 792 645
- US-A- 5 948 942
- US-A1- 2007 142 683

## Description

### Domaine technique

La présente invention concerne l'hydrogénation sélective de composés polyinsaturés présents dans une charge d'hydrocarbures comme par exemple dans les essences de vapocraquage. Ces essences contiennent en effet des composés générateurs de gommes, notamment des dioléfines et alkénylaromatiques, en mélange notamment avec des composés mono-oléfiniques et des composés aromatiques. Pour pouvoir valoriser les essences de vapocraquage, celles-ci ont besoin d'être débarrassées de leur teneur en dioléfines respectivement en alkénylaromatiques, les dioléfines étant hydrogénées sélectivement en mono-oléfines et les alkénylaromatiques étant hydrogénés sélectivement en aromatiques. De manière plus précise, l'invention se rapporte à un procédé de préparation d'un catalyseur supporté contenant du nickel et au moins un métal du groupe IB pour l'hydrogénation sélective des composés polyinsaturés présents dans les coupes hydrocarbures.

### Art antérieur

Les traitements par hydrogénation sélective sont généralement effectués sur des catalyseurs métalliques déposés sur un support amorphe ou cristallin. Les métaux utilisés sont les métaux du groupe VIII et parmi ceux-ci, on peut noter le nickel qui est d'un usage courant.

Cependant les catalyseurs au nickel ne sont pas suffisamment sélectifs car ils ont une tendance marquée à hydrogéner une part importante des mono-oléfines contenues dans la charge, même lorsque les hydrogénations sont effectuées à basses pressions, de l'ordre de 30 à 50 bar et à basse température, entre 50°C et 180°C (degrés Celsius).

Il est connu que l'amélioration de la sélectivité de ces catalyseurs peut être obtenue en injectant des composés sulfurés avant la mise en contact du catalyseur avec la charge réactive de manière à obtenir un catalyseur passivé au soufre. Ces composés peuvent être choisis parmi les composés suivants: thiophène, thiophane, alkylmonosulfures tels que diméthylsulfure, diéthylsulfure, dipropylsulfure, propylméthyl-sulfure. Cette sulfuration est cependant délicate à réaliser car il est nécessaire que le composé sulfuré soit très également réparti sur l'ensemble du lit catalytique pour que l'on puisse effectivement obtenir un effet marqué sur la sélectivité. De plus cette procédure est coûteuse et longue, ce qui se traduit en perte de production.

Le manque de sélectivité intrinsèque du catalyseur au nickel ne se manifeste pas seulement vis-à-vis des mono-oléfines, il se manifeste également vis-à-vis des aromatiques. Si la surface des particules de Ni était passivée en surface par des composés organiques soufrés de manière très contrôlée, a *priori* sur des sites de Ni bien distincts, et à raison d'un atome de S par environ 4 ou 5 atomes de Ni de surface [T.E. Fischer, S.R. Kelemen, J. Catal., 53, 24, (1978), ou GB1565754], l'hydrogénation des noyaux aromatiques serait supprimée et l'hydrogénation des composés oléfiniques serait fortement ralentie. La maîtrise à l'échelle industrielle du dépôt de soufre aussi contrôlé, ainsi que la stabilité de ce système dans des conditions réactionnelles utilisées pour les hydrogénations sélectives (25 - 30 bar, 50 - 180°C), est toutefois difficile à atteindre. C'est ainsi qu'au démarrage d'un catalyseur au nickel, même passivé par un composé sulfuré du type de ceux indiqués ci-dessus, il est nécessaire d'utiliser une charge de démarrage non réactive, ne contenant ni dioléfine, ni mono-oléfine et très peu d'aromatiques. En effet l'hydrogénation des dioléfines ou éventuellement des mono-oléfines sur les catalyseurs neufs qui sont très actifs, provoque un dégagement de chaleur suffisant pour élever la température du catalyseur à des niveaux bien supérieurs à 200°C, ce qui peut provoquer l'hydrogénation des aromatiques. Cette dernière réaction est encore plus exothermique et la température peut dépasser 600°C, ce qui a pour résultat de provoquer le craquage des hydrocarbures, une réaction elle-aussi très exothermique. C'est ainsi que les températures atteintes lors de ces emballements peuvent dépasser les températures de calcul des réacteurs en acier, ce qui oblige à remplacer non seulement la charge de catalyseur mais aussi le réacteur lui-même.

Une autre méthode d'amélioration de la sélectivité catalytique consiste à pré-passiver le catalyseur au Ni à l'état oxydé et en l'absence d'hydrogène, par imprégnation avec un composé organique du soufre, par exemple le 2,2 dithio bis éthanol (D.E.O.D.S), tel que décrit dans le brevet EP0466567. Le polysulfure se décompose sous hydrogène dans le réacteur d'hydrogénation, simultanément à la réduction du nickel, et l'introduction d'une charge réactive peut se faire par la suite sans danger d'emballement de la réaction. Toutefois, étant donné que la réduction et la passivation au soufre ont lieu en même temps, il est encore plus difficile dans ces conditions d'obtenir une passivation homogène de la surface du Ni avec la stoechiométrie souhaitée et uniquement sur les sites de Ni souhaités. Pour éviter la présence de particules de Ni réduit non passivé, le polysulfure est introduit en excès et une sur-passivation avec formation partielle de la phase Ni₃S₂ ne peut, en général, pas être évitée, ce qui diminue de façon significative l'activité catalytique même envers les dioléfines (B.W. Hoffer, R.L.C. Bonne, A.D. van Langeveld, C. Griffiths, C.M. Lok, J.A. Moulijn, Fuel 83 (2004) 1-8).

Il est également connu que les catalyseurs bimétalliques peuvent apporter des gains de sélectivité et de stabilité. Pour des catalyseurs d'hydrogénation sélective, il est connu que des associations du palladium avec un deuxième métal rendent les catalyseurs plus sélectifs mais moins actifs. De telles associations ont seulement été proposées pour les réactions d'hydrogénations sélectives des coupes contenant des hydrocarbures ayant entre deux et quatre atomes de carbone, pour lesquelles souvent un premier réacteur travaille avec un catalyseur monométallique au palladium pour effectuer la majeur partie de la conversion, et un deuxième réacteur contenant un catalyseur bimétallique complète la conversion de manière plus sélective. Par exemple, le brevet US5356851 enseigne qu'il est avantageux d'associer un métal du groupe VIII (préférentiellement le palladium) à un élément tel que l'indium ou le gallium pour des applications en hydrogénation sélective de composés polyinsaturés. De même des associations Pd-Cu (US5464802), Pd-Ag (US4547600), Pd-Sn et Pd-Pb (JP59227829) ou encore une combinaison de palladium et d'un métal alcalin (EP0722776) ont été identifiées pour leur performances en hydrogénation. L'ensemble de ces brevets vise une augmentation du rendement en mono-oléfines.

Des catalyseurs bimétalliques à base de nickel sont aussi décrits dans l'art antérieur. Le brevet GB1565754 décrit l'introduction de 10ppm d'un élément du groupe du platine, tel que le ruthénium, le rhodium, le palladium, l'osmium, l'iridium et le platine, et de façon préférée de palladium, dans un catalyseur d'hydrogénation sélective de coupes C3, C4 ou essence contenant 10% de Ni supporté sur sépiolite. Cette introduction a pour effet d'augmenter la réductibilité du Ni après régénération, mais l'introduction ultérieure de composés sulfures pour la passivation est toujours nécessaire. Le brevet US5997835 décrit l'introduction d'or dans un catalyseur au nickel supporté par une méthode en phase aqueuse pour le vaporeformage du méthane, l'effet de l'introduction de l'or étant de ralentir la désactivation du catalyseur.

Le brevet FR2792645 décrit la préparation en phase aqueuse à pH inférieur à 10 d'un catalyseur bimétallique associant un métal du groupe VIII (préférentiellement le platine, le palladium et le nickel) et un métal préférentiellement choisi parmi le germanium, l'étain, l'argent et l'or, pour l'hydrogénation sélective de composés polyinsaturés, dans un but d'augmenter le rendement en oléfines. Le brevet US5208405 décrit un procédé d'hydrogénation sélective des dioléfines contenant entre 4 et 10 atomes de carbone, ledit procédé minimise la formation de paraffines ainsi que la désactivation du catalyseur, lequel est un catalyseur supporté à base de nickel et d'argent, préparé de préférence par imprégnation d'un composé du nickel et d'un composé de l'argent de façon simultanée ou successive. Le rapport atomique Ag/Ni est compris entre 1 et 8.

Le brevet US5948942 enseigne qu'il est avantageux d'associer un métal complètement réduit du groupe VIII avec un métal partiellement réduit du groupe IB préférentiellement le cuivre, pour l'hydrogénation sélective et simultanée des dioléfines et des nitriles. Ces catalyseurs sont préparés préférentiellement par imprégnation successive des différentes solutions aqueuses de sels métalliques, avec calcination et activation intermédiaires. Le brevet US6417419 décrit un catalyseur supporté, pour l'hydrogénation sélective du butadiène, à base de cuivre et d'un métal activateur choisi parmi le nickel, le cobalt, le platine, le palladium, ou le manganèse dans lequel au moins 50 % poids des métaux sont répartis sur la couche externe du support d'une épaisseur de 200 microns. La méthode de préparation est l'imprégnation, la coprécipitation, la cogellation ou l'échange d'ion, de façon préférée, l'imprégnation d'une solution de sels de ces métaux. L'intérêt est d'améliorer la stabilité du catalyseur.

La présente invention a pour objectif de fournir un catalyseur contenant une phase métallique à base de nickel et d'au moins un métal du groupe IB, préparé par un nouveau procédé de préparation, pour l'hydrogénation sélective de composés polyinsaturés présents dans une coupe hydrocarbonée. Plus précisément, elle se propose de fournir un catalyseur alternatif aux catalyseurs à base de nickel passivés au soufre connus de l'art antérieur.

### Résumé et intérêt de l'invention

La présente invention a pour objet un procédé de préparation d'un catalyseur comprenant au moins un support poreux et au moins une phase métallique contenant du nickel et au moins un métal M du groupe IB dans une proportion telle que le rapport molaire M/Ni est compris entre 0,005 et 0,5, ledit procédé comprenant au moins successivement les étapes suivantes :
a1) le dépôt de nickel sur au moins ledit support pour obtenir un catalyseur supporté monométallique à base de nickel et,
b1) le dépôt, en présence d'au moins un gaz réducteur et en l'absence de tout solvant aqueux, d'au moins un composé organométallique d'au moins dudit métal M sur ledit catalyseur monométallique.

La présente invention a également pour objet un procédé d'hydrogénation sélective d'une charge d'hydrocarbures polyinsaturés, ledit procédé comprenant le passage de ladite charge dans au moins une unité réactionnelle pourvue d'au moins un catalyseur supporté comprenant au moins une phase métallique contenant du nickel et au moins un métal M du groupe IB préparé selon le procédé de préparation de l'invention.

Le catalyseur, préparé selon le procédé de l'invention et mis en oeuvre dans un procédé d'hydrogénation sélective d'hydrocarbures polyinsaturés, conduit à des performances catalytiques améliorées en terme de sélectivité envers les composés mono-insaturés. De plus, le catalyseur, préparé selon le procédé de l'invention et mis en oeuvre dans un procédé d'hydrogénation sélective d'hydrocarbures polyinsaturés, limite sensiblement voire supprime l'hydrogénation des noyaux aromatiques présents dans des composés polyinsaturés tels que les composés aromatiques, styréniques et indéniques, ce qui permet d'éviter tout emballement des réactions et de valoriser les composés présentant un noyau aromatique, issus dudit procédé d'hydrogénation sélective selon l'invention, dans diverses applications.

### Description de l'invention

La présente invention a pour objet un procédé de préparation d'un catalyseur comprenant au moins un support poreux et au moins une phase métallique contenant du nickel et au moins un métal M du groupe IB dans une proportion telle que le rapport molaire M/Ni est compris entre 0,005 et 0,5, ledit procédé comprenant au moins successivement les étapes suivantes :
a1) le dépôt de nickel sur au moins ledit support pour obtenir un catalyseur supporté monométallique à base de nickel et,
b1) le dépôt, en présence d'au moins un gaz réducteur et en l'absence de tout solvant aqueux, d'au moins un composé organométallique d'au moins dudit métal M sur ledit catalyseur monométallique.

Conformément à l'invention, le catalyseur préparé selon le procédé de l'invention comprend une phase métallique contenant du nickel et au moins un métal M du groupe IB choisi parmi le cuivre, l'argent et l'or, lesdits métaux étant déposés sur un support poreux. La teneur en nickel dans ledit catalyseur est avantageusement comprise entre 1 % poids et 50 % poids, de manière préférée entre 5 % poids et 40 % poids et de manière encore plus préférée entre 8 % poids et 30 % poids de la masse dudit catalyseur. La teneur en métal M du groupe IB varie suivant la nature dudit métal : elle est avantageusement comprise entre 0,005 % poids et 30 % poids de la masse dudit catalyseur lorsque M est le cuivre et entre 0,01 % poids et 50 % poids de la masse dudit catalyseur lorsque M est l'argent ou l'or. Le rapport molaire M/Ni est généralement compris entre 0,005 et 0,5, de façon préférée entre 0,01 et 0,5, et de façon encore plus préférée entre 0,03 et 0,3. Ledit catalyseur préparé selon le procédé de l'invention peut comprendre un ou plusieurs métal(ux) M du groupe IB. De manière très préférée, ledit métal M du groupe IB est l'or.

Le support poreux présent dans le catalyseur préparé selon le procédé de l'invention comprend généralement au moins un oxyde réfractaire qui est avantageusement sélectionné parmi les oxydes de métaux des groupes 2, 3, 4, 13 et 14 de la nouvelle classification périodique des éléments tel que par exemple les oxydes de magnésium, d'aluminium, de silicium, de titane, de zirconium, de thorium pris seuls ou en mélange entre eux ou en mélange avec d'autres oxydes de métaux de la classification périodique. On peut aussi utiliser le charbon. Le support préféré est choisi parmi les alumines, les silices ou les silices-alumines, et de façon encore plus préférée il s'agit d'une alumine ou d'une silice. Le volume poreux du support est généralement compris entre 0,1 cm³/g et 1,5 cm³/g, de préférence compris entre 0,5 cm³/g et 1 cm³/g. La surface spécifique du support est généralement comprise entre 10 m²/g et 250 m²/g, de préférence entre 30 m²/g et 200 m²/g et de façon encore plus préférée entre 40 m²/g et 180 m²/g. Ledit support poreux se présente avantageusement sous forme de billes, d'extrudés, de pastilles, ou d'agglomérats irréguliers et non sphériques dont la forme spécifique peut résulter d'une étape de concassage. De manière très avantageuse, ledit support se présente sous forme de billes ou d'extrudés.

Conformément à l'étape a1) du procédé de préparation selon l'invention, le nickel est déposé sur le support poreux. Le dépôt du nickel sur ledit support peut être effectué par toute méthode connue de l'homme du métier. Par exemple, le dépôt est réalisé par imprégnation consistant en la mise en contact dudit support poreux avec au moins une solution aqueuse ou organique d'au moins un composé du nickel ou avec une suspension d'au moins un composé organique ou inorganique du nickel, ou bien le dépôt est réalisé par des méthodes de déposition - précipitation bien connues de l'homme du métier [J.W. Geus, Preparation of Catalysts III, dans G. Poncelet, P. Grange, P.A. Jacobs (Eds.), Elsevier, Amsterdam 1983, 1]. Le dépôt du nickel sur le support est suivi éventuellement par un ou plusieurs lavages et/ou éventuellement par une évaporation du solvant. De manière avantageuse, le dépôt du nickel sur le support poreux est suivi d'un ou plusieurs traitements thermiques ou chimiques conduisant à un catalyseur supporté monométallique à base de nickel à l'état majoritairement oxyde ou majoritairement métallique. Ladite étape a1) conduit à la préparation d'un catalyseur supporté monométallique à base de Ni.

Conformément à l'étape b1) du procédé de préparation selon l'invention, on procède à au moins une étape de dépôt, en présence d'au moins un gaz réducteur et en l'absence de tout solvant aqeux, d'au moins un composé organométallique d'au moins dudit métal M sur ledit catalyseur monométallique à base de Ni. Le gaz réducteur est préférentiellement de l'hydrogène. Ladite étape b1) est effectuée à une température comprise entre 10°C et 100°C, de préférence comprise entre 20°C et 50°C et pendant une durée comprise entre 10 minutes et 24 heures. Selon un mode de mise en oeuvre préférée, ladite étape b1) est effectuée en phase gazeuse. Selon un mode de mise en oeuvre encore plus préférée, ladite étape b1) est effectuée en phase liquide dans un solvant organique, par exemple l'heptane ou le toluène.

Le(s) métal(ux) M du groupe IB, de préférence l'or, est(sont) introduit(s) sous la forme d'un composé organométallique de ce(s) métal(ux). Ledit composé organométallique d'au moins dudit métal M employé pour la mise en oeuvre de ladite étape b1) comprend au moins une liaison carbone-métal M (liaison C-M), de manière préférée une liaison carbone-or.

Tous les composés organométalliques de l'or comprenant au moins une liaison C-Au conviennent pour la mise en oeuvre de ladite étape b1). On utilise avantageusement le cyanure d'or [AuCN], l'or diméthyle acétyle acétonate [Au(CH₃)₂(acac)], l'or diméthyle iode dimère [((CH₃)₂Aul)₂], le diméthyle or carboxylate [(CH₃)₂Au(COOR) avec R=CH₃ ou tertiobutyl], le triphénylphosphine chlorure d'or [Ph₃PAuCl], le diméthyle or oxinate [(CH₃)₂AuL avec L=8-quinolénol]. De manière préférée, le composé organométallique de l'or est choisi parmi le cyanure d'or [AuCN], l'or acétyle acétonate [Au(CH₃)₂(acac)] et l'or diméthyle iode dimère [((CH₃)₂Aul)₂] et de façon encore plus préférée, il s'agit du cyanure d'or [AuCN].

Tous les composés organométalliques de l'argent comprenant au moins une liaison C-Ag conviennent pour la mise en oeuvre de ladite étape b1). On utilise avantageusement le cyanure d'argent [AgCN], l'argent acétyle acétonate [Ag(acac)], le N,N'-diisopropylacétamidinato argent [(Ag (iPr-Me-AMD))x (x=2,3)], le trans-bis(triméthylsilyl)éthène(1,1,1,5,5,5-hexafluoro-2,4-pentanedionato) argent, l'argent éthylhexanoate [AgOOCCH(C₂H₅)C₄H₉]. Les composés organométalliques de l'argent préférés sont le cyanure d'argent [AgCN] et l'argent acétyle acétonate [Ag(acac)].

Tous les composés organométalliques du cuivre comprenant au moins une liaison C-Cu conviennent pour la mise en oeuvre de ladite étape b1). On utilise avantageusement le cyanure de cuivre [CuCN], le cuivre acétyle acétonate [Cu(acac)], le (N,N'-diisopropylacétamidinato) cuivre [(Cu(iPr-Me-AMD))_{2]}, le N,N'-diisopropyl-2-nbutylamidinato cuivre [(Cu(iPr-nBu-AMD))₂], le cuivre hexafluoro-acétylacétonato [Cu(hfac)2], le cyclopentadiényl cuivre triéthyle phosphine, le cyclopentadiényl cuivre tertiarybutylisocyanide, l'éthylhexanoate de cuivre [Cu(OOCCH(C₂H₅)C₄H₉)₂], le bis(2,2,6,6-tétraméthyl-3,5-heptanedionate) cuivre [Cu(OCC(CH₃)₃CHCOC(CH₃)₃)₂]. Les composés organométalliques du cuivre préférés sont le cyanure de cuivre [CuCN], le cuivre acétyle acétonate [Cu(acac)].

L'ensemble des composés organométalliques cités ci-dessus et leurs méthodes de préparation sont connus de I'Homme du métier. Lesdits composés sont soit commercialisés soit décrits dans la littérature.

Selon un premier mode de réalisation particulier du procédé de préparation selon l'invention, l'étape a1) est suivie d'une étape a2), préalablement à la mise en oeuvre de ladite étape b1), ladite étape a2) consistant à procéder à la réduction dudit catalyseur monométallique obtenu à l'issue de ladite étape a1) en présence d'au moins un gaz réducteur, préférentiellement l'hydrogène. Plus précisément, ladite étape a2) consiste généralement en une montée lente de température, par exemple comprise entre 0,1°C/minute et 5°C/minute, sous courant de gaz réducteur, de préférence sous hydrogène, jusqu'à la température maximale de réduction, comprise entre 100°C et 600°C, de manière préférée entre 200°C et 500°C, suivie d'un maintien à cette température pendant une durée comprise entre 1 heure et 40 heures, de manière préférée entre 5 heures et 30 heures. Ces conditions permettent d'obtenir un catalyseur supporté monométallique très majoritairement réduit.

Selon un deuxième mode de réalisation particulier du procédé de préparation selon l'invention, ladite étape b1) est suivie d'au moins une étape c1) au cours de laquelle ledit catalyseur supporté contenant ladite phase métallique à base de nickel et d'au moins un métal du groupe IB, obtenu à l'issue de ladite étape b1), est soumis à au moins une phase de séchage. Ladite étape c1) est effectuée sous vide, sous courant de gaz inerte (azote, argon, hélium) ou d'air, de préférence sous vide ou sous courant de gaz inerte. Elle est menée à une température comprise entre 10°C et 150°C, de préférence comprise entre 20°C et 50°C.

Selon un troisième mode de réalisation particulier du procédé de préparation selon l'invention, ladite étape b1) est suivie d'au moins une étape c2) au cours de laquelle ledit catalyseur supporté contenant ladite phase métallique à base de nickel et d'au moins un métal du groupe IB, obtenu à l'issue de ladite étape b1), est soumis à au moins une phase de lavage. Le(s) lavage(s) est(sont) préférentiellement réalisé(s) au moyen d'un hydrocarbure, par exemple celui qui a été éventuellement utilisé comme solvant dans ladite étape b1) lorsque celle-ci est mise en oeuvre en phase liquide.

Conformément au procédé de préparation selon l'invention, lesdits deuxième et troisième modes de réalisation particuliers décrits ci-dessus sont indépendants l'un de l'autre. De manière avantageuse, ledit procédé de préparation selon l'invention met en oeuvre au moins ladite étape c1) et au moins ladite étape c2), lesdites étapes c1) et c2) étant effectuées à la suite de ladite étape b1) dans un ordre indifférent. De préférence, le catalyseur issu de ladite étape b1) est soumis à au moins une étape de séchage selon c1) puis au moins une étape de lavage selon c2). Une nouvelle étape de séchage selon c1) est souvent également mise en oeuvre à la suite d'une étape de lavage selon c2).

Selon un quatrième mode de réalisation particulier du procédé de préparation selon l'invention, ladite étape b1) est suivie d'une étape d1) consistant à procéder à l'activation du catalyseur obtenu à l'issue de ladite b1) en présence d'au moins un gaz réducteur, préférentiellement l'hydrogène. Ladite étape d1) est effectuée à une température comprise entre 150°C et 600°C, de manière préférée entre 200°C et 500°C, de manière encore plus préférée entre 300°C et 500°C, pendant une durée comprise entre 1 minute et 30 heures, de manière préférée entre 10 minutes et 10 heures. La montée jusqu'à cette température d'activation est généralement lente, par exemple comprise entre 0,1 °C/minute et 5°C/minute. Cette activation en présence d'un gaz réducteur peut s'effectuer soit de façon statique soit sous courant de gaz réducteur, de préférence sous courant de gaz réducteur.

Conformément au procédé de préparation selon l'invention, un ou plusieurs modes de réalisation particuliers décrits ci-dessus peuvent être effectués dans le déroulement dudit procédé. D'une manière préférée, on met en oeuvre successivement les étapes a1), b1), c1), c2) et d1) et d'une manière encore plus préférée on met en oeuvre successivement les étapes a1), a2), b1), c1), c2) et d1).

Le catalyseur obtenu à l'issue de la mise en oeuvre de ladite étape b1), ou à l'issue de la mise en oeuvre de ladite étape c1) et/ou de ladite étape c2) ou bien encore à l'issue de la mise en oeuvre de ladite étape d1) peut être utilisé directement dans une unité réactionnelle réalisant la conversion d'une charge hydrocarbonée, en particulier dans une unité réactionnelle réalisant l'hydrogénation sélective d'une charge d'hydrocarbures polyinsaturés. Ledit catalyseur préparé selon le procédé de l'invention peut aussi être stocké à l'air puis réduit avant utilisation. La réduction consiste alors généralement en une montée lente de température, par exemple comprise entre 0,1°C/minute et 5°C/minute, sous courant de gaz réducteur, de préférence sous hydrogène, jusqu'à la température maximale de réduction, comprise entre 100°C et 600°C, de manière préférée entre 200°C et 500°C, suivie d'un maintien à cette température pendant une durée comprise entre 1 heure et 40 heures, de manière préférée entre 5 heures et 30 heures.

La présente invention a également pour objet un procédé d'hydrogénation sélective d'une charge d'hydrocarbures polyinsaturés, ledit procédé comprenant le passage de ladite charge dans au moins une unité réactionnelle pourvue d'au moins un catalyseur supporté comprenant au moins une phase métallique contenant du nickel et au moins un métal M du groupe IB préparé selon le procédé de préparation de l'invention.

Ladite charge d'hydrocarbures polyinsaturés, traitée dans le procédé d'hydrogénation sélective selon l'invention, est avantageusement une essence de vapocraquage comportant des hydrocarbures polyinsaturés contenant au moins 4 atomes de carbone et ayant un point d'ébullition final allant jusqu'à 220°C. Plus précisément, lesdits hydrocarbures polyinsaturés présents dans la charge traitée selon le procédé d'hydrogénation sélective de l'invention sont en particulier des composés dioléfiniques, des composés styréniques et des composés indéniques. Parmi les composés dioléfiniques, ladite charge contient notamment du butadiène, de l'isoprène et du cyclopentadiène. Parmi les composés styréniques, ladite charge contient notamment du styrène et de l'alpha-méthylstyrène. Parmi les composés indéniques, ladite charge contient notamment de l'indène.

Le procédé d'hydrogénation sélective selon l'invention vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants.

L'effluent obtenu après la mise en oeuvre du procédé d'hydrogénation sélective de l'invention présente une teneur sensiblement amoindrie en hydrocarbures polyinsaturés, en particulier il présente une teneur amoindrie en composés dioléfiniques, en composés styréniques et en composés indéniques, tout en conservant une teneur en composés aromatiques (plus précisément une teneur en noyaux aromatiques) proche de celle présente dans ladite charge d'hydrocarbures. Ledit effluent est avantageusement valorisable comme base dans une essence ou utilisable comme base pour la valorisation de composés aromatiques.

Le procédé d'hydrogénation sélective selon l'invention est avantageusement effectué sous pression, en phase liquide, en présence d'une quantité d'hydrogène en faible excès par rapport à la valeur stoechiométrique permettant l'hydrogénation sélective des composés polyinsaturés présents dans la charge d'hydrocarbures, c'est-à-dire un excès compris généralement entre 5 et 30%. Le procédé d'hydrogénation sélective selon l'invention est mis en oeuvre à une température comprise entre 20°C et 200°C. La pression est généralement suffisante pour maintenir au moins 80 % poids de la charge à traiter en phase liquide à l'entrée de l'unité réactionnelle. Elle est généralement comprise entre 0,4 MPa et 5 MPa, plus avantageusement entre 1 MPa et 4 MPa. La vitesse spatiale horaire (définie comme le rapport du débit volumique de charge au volume de catalyseur), établie dans ces conditions est généralement comprise entre 0,2 h⁻¹ et 30 h⁻¹ et de préférence entre 1 h⁻¹ et 20 h⁻¹, et de manière encore plus préférée, entre 2 h⁻¹ et 10 h⁻¹.

La mise en oeuvre technologique du procédé d'hydrogénation sélective est par exemple réalisée par injection, en courant ascendant ou descendant, de la charge et de l'hydrogène dans un réacteur à lit fixe. Elle peut également être avantageusement réalisée par l'implantation d'au moins dudit catalyseur supporté contenant du nickel et au moins un métal du groupe IB dans une colonne de distillation réactive ou dans des réacteurs - échangeurs.

Les exemples qui suivent illustrent l'invention sans en limiter la portée.

### Exemple 1 (comparatif): préparation d'un catalyseur A monométallique supporté sur silice et contenant du nickel

Un catalyseur de type Ni / silice (catalyseur A) est préparé par échange cationique du sel [Ni(NH₃)₆]²⁺ sur la silice. La solution d'imprégnation est préparée en mélangeant une solution de nitrate de nickel de concentration 7.10⁻³ mol/L à une solution ammoniacale de concentration 0,4 mol/L. Une quantité de 4 g de silice (Aerosil-200, Degussa) est mise en contact avec la solution d'imprégnation durant 24 heures, à température ambiante et sous agitation. Le solide est ensuite récupéré par filtration, lavé avec de l'eau permutée, séché à l'étuve sous vide à 80°C puis 100°C et enfin traité sous courant d'hydrogène à 500°C avant d'être refroidi et conservé à l'air.

Le catalyseur monométallique A ainsi obtenu contient 11,7 % poids de Ni (d'après l'analyse élémentaire).

### Exemple 2 (selon l'invention) : préparation d'un catalyseur B supporté sur silice et contenant du nickel et de l'or

Une quantité de 40 mg du catalyseur monométallique A est préalablement réduite sous courant d'hydrogène à 400°C durant 14 heures. Un catalyseur B est préparé en mettant en contact 40 mg du catalyseur monométallique A préalablement réduit avec une suspension de cyanure d'or (AuCN, Strem) dans l'heptane (Acros) (4 mg de AuCN dans 20 mL de n-heptane), à température ambiante, en présence d'hydrogène et sous agitation (agitateur magnétique avec barreau aimanté). Après 12 heures de mise en contact, le catalyseur B ainsi obtenu est directement introduit dans l'autoclave en vue du test d'hydrogénation sélective du styrène illustré dans l'exemple 6.

### Exemple 3 (selon l'invention) : préparation d'un catalyseur C supporté sur silice et contenant du nickel et de l'or

Une quantité de 1,08 g du catalyseur monométallique A est préalablement réduite sous courant d'hydrogène à 400°C durant 14 heures. Un catalyseur C est préparé en mettant en contact 1,08 g du catalyseur monométallique A préalablement réduit avec une suspension de cyanure d'or (AuCN, Strem) dans l'heptane (Acros) (100 mg de AuCN dans 20 mL de n-heptane), à température ambiante, en présence d'hydrogène et sous agitation (agitateur magnétique avec barreau aimanté). Après 12 heures de mise en contact, le solide est séché sous vide (10⁻¹ mbar), lavé 4 fois par 20 mL de n-heptane et remis sous vide (10⁻¹ mbar) à température ambiante. Il est ensuite stocké sous argon (1 atm).

Le catalyseur C ainsi obtenu contient 11,5 % poids de nickel et 9,6 % poids d'or (d'après l'analyse élémentaire), ce qui correspond à un rapport molaire Au / Ni égal à 0,25.

### Exemple 4 (selon l'invention): préparation d'un catalyseur D supporté sur silice et contenant du nickel et de l'or

Une quantité de 1,08 g du catalyseur monométallique A est préalablement réduit sous courant d'hydrogène à 400°C durant 14 heures. Un catalyseur D est préparé en mettant en contact 1,08 g du catalyseur monométallique A préalablement réduit avec une suspension de cyanure d'or (AuCN, Strem) dans l'heptane (Acros) (100 mg de AuCN dans 20 mL de n-heptane), à température ambiante, en présence d'hydrogène et sous agitation (agitateur magnétique avec barreau aimanté). Après 12 heures de mise en contact, le solide est séché sous vide (10⁻¹ mbar), lavé 4 fois par 20 mL de n-heptane, remis sous vide (10⁻¹ mbar) à température ambiante et stocké sous argon (1 atm). Il est ensuite mis en présence d'hydrogène (100 mbar) et porté à 400°C pendant 2 heures (rampe de température de 1,5°C/minute). Enfin, il est stocké sous argon à température ambiante.

Le catalyseur D ainsi obtenu contient 11,5 % poids de nickel et 9,6 % poids d'or (d'après l'analyse élémentaire), ce qui correspond à un rapport molaire Au / Ni égal à 0,25.

### Exemple 5 (comparatif): préparation d'un catalyseur E supporté sur silice et contenant du nickel et de l'or en utilisant un sel métallique de l'or comme précurseur.

Une solution aqueuse du sel [Au(NH₃)₄]³⁺ est préalablement préparée en dissolvant 31 mg de Au(NH₃)₄(NO₃)₃ dans 20 mL d'eau distillée. Le complexe Au(NH₃)₄(NO₃)₃ est lui-même préalablement préparé en dissolvant de l'acide chloroaurique HAuCl₄ (Aldrich) dans une solution aqueuse de nitrate d'ammonium (Aldrich) ; de l'ammoniaque concentré est ajouté goutte à goutte de telle sorte que le pH soit toujours inférieur à 5. Le complexe ainsi formé est lavé avec de l'eau et de l'éther, séché sous vide puis conservé à l'air.

Le catalyseur monométallique A est préalablement réduit sous courant d'hydrogène à 400°C durant 14 heures. Un catalyseur E est préparé en mettant en contact 0,5 g du catalyseur A préalablement réduit avec 20 mL d'une solution aqueuse du sel [Au(NH₃)₄]³⁺, pendant 5 heures, à température ambiante, en présence d'hydrogène et sous agitation. Le solide est ensuite récupéré par filtration, lavé avec de l'éthanol puis de l'heptane, séché sous vide puis à l'air à température ambiante puis traité sous courant d'hydrogène à 400°C.

Le catalyseur E ainsi obtenu contient 11,6 %poids de nickel et 2,6 % poids d'or (d'après l'analyse élémentaire).

### Exemple 6 : Performances catalytiques des catalyseurs A à E en hydrogénation du styrène.

Les propriétés catalytiques des catalyseurs préparés selon les exemples ci-dessus sont évaluées successivement dans un procédé d'hydrogénation du styrène. L'hydrogénation sélective du styrène conduit à l'éthylbenzène, cette hydrogénation constituant la réaction désirée. L'hydrogénation totale du styrène conduit à l'éthylcyclohexane, lequel est produit par une réaction successive indésirable.

L'hydrogénation du styrène est réalisée dans un autoclave de 100mL en acier inoxydable, muni d'une agitation mécanique à entraînement magnétique et pouvant fonctionner sous une pression maximale de 100 bar et des températures comprises entre 5°C et 200°C.

Une quantité de catalyseur (40 mg) est transvasée dans l'autoclave, à l'abri de l'air. Le catalyseur monométallique A est préalablement réduit sous courant d'hydrogène à 400°C durant 14 heures. Les autres catalyseurs B, C, D et E sont utilisés tel quel. Après ajout de 50 mL de n-heptane, l'autoclave est fermé, purgé, puis pressurisé sous 15 bar (0,15 MPa) d'hydrogène. Avant introduction du styrène, il est porté à 150°C pendant 2 heures sous agitation puis ramené à la température initiale du test (5°C). Au temps t=0, 3,5 g de styrène et un étalon interne sont introduits dans l'autoclave et l'agitation (500 tr/min) est mise en route. L'avancement de la réaction est suivi par prélèvement d'échantillons du milieu réactionnel à intervalles de temps réguliers. Ces échantillons sont analysés par chromatographie en phase gazeuse. Une fois que tout le styrène a été consommé, la pression dans l'autoclave est portée à 60 bar et la température est portée rapidement à 150°C (en moins de 1 heure) : dans cette deuxième étape, la réaction qui a lieu est l'hydrogénation de l'éthylbenzène en éthylcyclohexane.

Les performances des catalyseurs sont évaluées en terme de sélectivité, laquelle est évaluée à partir de la mesure des vitesses de réaction des réactions d'hydrogénation. Les vitesses de réaction des deux réactions d'hydrogénation du styrène en éthylbenzène et d'hydrogénation de l'éthylbenzène en éthylcyclohexane sont définies comme les pentes à l'origine rapportées à la masse de catalyseur des courbes de l'évolution de la concentration en éthylbenzène au cours du temps. La vitesse de réaction r1 de l'hydrogénation du styrène en éthylbenzène est déterminée sur la première étape du test. La vitesse de réaction r2 de l'hydrogénation de l'éthylbenzène en éthylcyclohexane est déterminée sur la deuxième étape du test. La sélectivité du catalyseur envers le produit désiré, à savoir l'éthylbenzène, est évaluée par le rapport r1 / r2 ; le catalyseur est d'autant plus sélectif envers l'éthylbenzène que ce rapport est plus élevé.

Les sélectivités des catalyseurs préparés selon les exemples ci-dessus sont comparées dans le tableau 1 à celle du catalyseur monométallique au Ni (catalyseur A).

**Tableau 1 : sélectivités des catalyseurs supportés sur silice. Comparaison par rapport au catalyseur A monométallique à base de nickel seulement.**

| **Catalyseur** | **Sélectivité** **(r1/r2) / (r1_{ref}/r2_{ref})** |
|---|---|
| A | 1 |
| B | 3,2 |
| C | 3,1 |
| D | 5,9 |
| E | 1,0 |

Dans le tableau 1, r1ᵣₑₜ respectivement r2_{ref} correspond à la vitesse de réaction r1 définie plus haut, respectivement à la vitesse de réaction r2 définie plus haut, l'une et l'autre étant mesurée avec le catalyseur monométallique A.

Les catalyseurs B, C et D à base de nickel et d'or, préparés selon le procédé de l'invention, présentent une sélectivité 3 à 6 fois plus élevée que celle du catalyseur monométallique au nickel (catalyseur A). Pour ces catalyseurs B, C et D à base de nickel et d'or, la vitesse d'hydrogénation de l'éthylbenzène en éthylcyclohexane est donc fortement ralentie par rapport à la vitesse d'hydrogénation du styrène en éthylbenzène. Pour lesdits catalyseurs B, C et D, l'hydrogénation du cycle aromatique est donc sensiblement ralentie par rapport à celle observée avec le catalyseur A, lequel favorise la production d'éthylcyclohexane au détriment de l'éthylbenzène. Les catalyseurs B, C et D sont donc bien plus sélectifs envers l'éthylbenzène que le catalyseur A. L'ajout d'or permet donc de ralentir la réaction d'hydrogénation du noyau aromatique du styrène.

Les catalyseurs C et D, préparés selon un protocole identique à l'exception du dernier traitement d'activation (en présence d'hydrogène, à 400°C, pendant 2 heures) subi uniquement par le catalyseur D, illustrent l'intérêt de ce traitement d'activation. Il permet d'augmenter de façon significative la sélectivité du catalyseur envers l'éthylbenzène.

Les catalyseurs B, C et D, préparés selon le procédé de l'invention, sont également bien plus sélectifs que le catalyseur E, lequel a été préparé en présence d'un sel métallique de l'or en milieu aqueux.

## Revendications

1. Procédé de préparation d'un catalyseur comprenant au moins un support poreux et au moins une phase métallique contenant du nickel et au moins un métal M du groupe IB dans une proportion telle que le rapport molaire M/Ni est compris entre 0,005 et 0,5, ledit procédé comprenant au moins successivement les étapes suivantes :
a1) le dépôt de nickel sur au moins ledit support pour obtenir un catalyseur supporté monométallique à base de nickel et,
b1) le dépôt, en présence d'au moins un gaz réducteur et en l'absence de tout solvant aqueux, d'au moins un composé organométallique d'au moins dudit métal M sur ledit catalyseur monométallique.

2. Procédé de préparation selon la revendication 1 tel que ledit catalyseur présente un rapport molaire M/Ni compris entre 0,01 et 0,5.

3. Procédé de préparation selon la revendication 1 ou la revendication 2 tel que ledit support poreux présent dans ledit catalyseur est une alumine ou une silice.

4. Procédé de préparation selon l'une des revendications 1 à 3 tel que ledit métal M du groupe IB est l'or.

5. Procédé de préparation selon l'une des revendications 1 à 4 tel que ladite étape b1) est effectuée en phase gazeuse.

6. Procédé de préparation selon l'une des revendications 1 à 4 tel que ladite étape b1) est effectuée en phase liquide dans un solvant organique.

7. Procédé de préparation selon l'une des revendications 1 à 6 tel que ledit composé organométallique est le cyanure d'or lorsque ledit métal M est l'or.

8. Procédé de préparation selon l'une des revendications 1 à 7 tel que ladite étape a1) est suivie d'une étape a2), préalablement à la mise en oeuvre de ladite étape b1), ladite étape a2) consistant à procéder à la réduction dudit catalyseur monométallique en présence d'au moins un gaz réducteur.

9. Procédé de préparation selon l'une des revendications 1 à 8 tel que ladite étape b1) est suivie d'au moins une étape c1) au cours de laquelle ledit catalyseur supporté, obtenu à l'issue de ladite étape b1), est soumis à au moins une phase de séchage.

10. Procédé de préparation selon l'une des revendications 1 à 9 tel que ladite étape b1) est suivie d'au moins une étape c2) au cours de laquelle ledit catalyseur supporté, obtenu à l'issue de ladite étape b1), est soumis à au moins une phase de lavage.

11. Procédé de préparation selon l'une des revendications 1 à 10 tel que ladite étape b1) est suivie d'une étape d1) consistant à procéder à l'activation du catalyseur obtenu à l'issue de ladite b1) en présence d'au moins un gaz réducteur.

12. Procédé d'hydrogénation sélective d'une charge d'hydrocarbures polyinsaturés comprenant le passage de ladite charge dans au moins une unité réactionnelle pourvue d'au moins un catalyseur supporté comprenant au moins une phase métallique préparé selon le procédé de préparation selon l'une des revendications 1 à 11.

13. Procédé d'hydrogénation sélective selon la revendication 12 tel que ladite charge d'hydrocarbures polyinsaturés est une essence de vapocraquage comportant des hydrocarbures polyinsaturés contenant au moins 4 atomes de carbone et ayant un point d'ébullition final allant jusqu'à 220°C.

14. Procédé d'hydrogénation sélective selon la revendication 13 tel que lesdits hydrocarbures polyinsaturés sont des composés dioléfiniques, des composés styréniques et des composés indéniques.

15. Procédé d'hydrogénation sélective selon l'une des revendications 12 à 14 tel qu'il est mis en oeuvre à une température comprise entre 20°C et 200°C, sous une pression comprise entre 0,4 MPa et 5 MPa, avec une vitesse spatiale horaire (définie comme le rapport du débit volumique de charge au volume de catalyseur), comprise entre 0,2 h⁻¹ et 30 h⁻¹.

## Claims

1. A process for preparing a catalyst comprising at least one porous support and at least one metallic phase containing nickel and at least one metal M from group IB in a proportion such that the Molar ratio M/Ni is in the range 0.005 to 0.5, said process comprising at least the following steps in succession:
a1) depositing nickel on at least said support in order to obtain a supported nickel-based monometallic catalyst; and
b1) depositing, in the presence of at least one reducing gas and in the absence of any aqueous solvent, at least one organometallic compound of at least said metal M onto said monometallic catalyst.

2. A preparation process according to claim 1, in which said catalyst has a M/Ni molar ratio in the range 0.01 to 0.5.

3. A preparation process according to claim 1 or claim 2, in which said porous support present in said catalyst is an alumina or a silica.

4. A preparation process according to one of claims 1 to 3, in which said metal M from group IB is gold.

5. A preparation process according to one of claims 1 to 4, in which said step b1) is carried out in the gas phase.

6. A preparation process according to one of claims 1 to 4, in which said step b1) is carried out in the liquid phase in an organic solvent.

7. A preparation process according to one of claims 1 to 6, in which said organometallic compound is gold cyanide when said metal M is gold.

8. A preparation process according to one of claims 1 to 7, in which said step a1) is followed by a step a2) prior to carrying out said step b1), said step a2) consisting of carrying out a reduction of said monometallic catalyst in the presence of at least one reducing gas.

9. A preparation process according to one of claims 1 to 8, in which said step b1) is followed by at least one step c1) during which said supported catalyst obtained at the end of said step b1) undergoes at least one drying phase.

10. A preparation process according to one of claims 1 to 9, in which said step b1) is followed by at least one step c2) during which said supported catalyst obtained at the end of said step b1) undergoes at least one washing phase.

11. A preparation process according to one of claims 1 to 10, in which said step b1) is followed by a step d1) consisting of activating the catalyst obtained at the end of said step b1) in the presence of at least one reducing gas.

12. A process for the selective hydrogenation of a feed of polyunsaturated hydrocarbons, comprising passing said feed into at least one reaction unit provided with at least one supported catalyst comprising at least one metallic phase prepared in accordance with the preparation process according to one of claims 1 to 11.

13. A selective hydrogenation process according to claim 12, in which said feed of polyunsaturated hydrocarbons is a steam cracked gasoline comprising polyunsaturated hydrocarbons containing at least 4 carbon atoms and having an end point of up to 220°C.

14. A selective hydrogenation process according to claim 13, in which said polyunsaturated hydrocarbons are diolefin compounds, styrene compounds and indene compounds.

15. A selective hydrogenation process according to one of claims 12 to 14, carried out at a temperature in the range 20°C to 200°C, at a pressure in the range 0.4 MPa to 5 MPa, at an hourly space velocity (defined as the ratio of the volume flow rate of feed to the volume of catalyst) in the range 0.2 h⁻¹ to 30 h⁻¹.

## Patentansprüche

1. Verfahren zur Herstellung eines Katalysators, umfassend mindestens einen porösen Träger und mindestens eine metallische Phase, welche Nickel und mindestens ein Metall M der Gruppe IB in einem derartigen Anteil enthält, dass das Molverhältnis M/Ni im Bereich von 0,005 bis 0,5 liegt, wobei das Verfahren mindestens die nacheinander folgenden Schritte umfasst:
a1) Abscheiden von Nickel auf wenigstens dem Träger, um einen monometallischen geträgerten Katalysator auf Nickelbasis zu erhalten, und
b1) Abscheiden mindestens einer organometallischen Verbindung mindestens des Metalls M auf dem monometallischen Katalysator, in Gegenwart mindestens eines reduzierenden Gases und in Abwesenheit jedweden wässrigen Lösemittels.

2. Herstellungsverfahren nach Anspruch 1, derart, dass der Katalysator ein M/Ni-Molverhältnis im Bereich von 0,01 bis 0,5 aufweist.

3. Herstellungsverfahren nach Anspruch 1 oder Anspruch 2, derart, dass es sich bei dem porösen Träger, der in dem Katalysator vorliegt, um ein Aluminiumoxid oder ein Siliciumdioxid handelt.

4. Herstellungsverfahren nach einem der Ansprüche 1 bis 3, derart, dass es sich bei dem Metall M der Gruppe IB um Gold handelt.

5. Herstellungsverfahren nach einem der Ansprüche 1 bis 4, derart, dass der Schritt b1) in der Gasphase durchgeführt wird.

6. Herstellungsverfahren nach einem der Ansprüche 1 bis 4, derart, dass der Schritt b1) in der Flüssigphase in einem organischen Lösemittel durchgeführt wird.

7. Herstellungsverfahren nach einem der Ansprüche 1 bis 6, derart, dass es sich bei der organometallischen Verbindung um Goldcyanid handelt, wenn das Metall M Gold ist.

8. Herstellungsverfahren nach einem der Ansprüche 1 bis 7, derart, dass auf den Schritt a1) ein Schritt a2) folgt, bevor der Schritt b1) durchgeführt wird, wobei der Schritt a2) darin besteht, die Reduktion des monometallischen Katalysators in Gegenwart mindestens eines reduzierenden Gases durchzuführen.

9. Herstellungsverfahren nach einem der Ansprüche 1 bis 8, derart, dass auf den Schritt b1) mindestens ein Schritt c1) folgt, im Laufe dessen der geträgerte Katalysator, welcher nach Abschluss des Schrittes b1) erhalten wurde, mindestens einem Trocknungsschritt unterzogen wird.

10. Herstellungsverfahren nach einem der Ansprüche 1 bis 9, derart, dass auf den Schritt b1) mindestens ein Schritt c2) folgt, im Laufe dessen der geträgerte Katalysator, welcher nach Abschluss des Schrittes b1) erhalten wurde, mindestens einem Waschschritt unterzogen wird.

11. Herstellungsverfahren nach einem der Ansprüche 1 bis 10, derart, dass auf den Schritt b1) ein Schritt d1) folgt, welcher darin besteht, die Aktivierung des Katalysators, der nach Abschluss des Schrittes b1) erhalten wurde, in Gegenwart mindestens eines reduzierenden Gases durchzuführen.

12. Verfahren zur selektiven Hydrierung einer Charge mehrfach ungesättigter Kohlenwasserstoffe, wobei es das Einleiten der Charge in mindestens eine Reaktionseinheit umfasst, welche mit mindestens einem geträgerten Katalysator versehen ist, der mindestens eine metallische Phase umfasst, die gemäß dem Herstellungsverfahren nach einem der Ansprüche 1 bis 11 hergestellt ist.

13. Verfahren zur selektiven Hydrierung nach Anspruch 12, derart, dass es sich bei der Charge mehrfach ungesättigter Kohlenwasserstoffe um ein Benzin aus dem Dampfkrackverfahren handelt, welches mehrfach ungesättigte Kohlenwasserstoffe aufweist, die mindestens 4 Kohlenstoffatome enthält und einen Endsiedepunkt von bis zu 220 °C hat.

14. Verfahren zur selektiven Hydrierung nach Anspruch 13, derart, dass es sich bei den mehrfach ungesättigten Kohlenwasserstoffen um diolefinische Verbindungen, styrolartige Verbindungen und um indenartige Verbindungen handelt.

15. Verfahren zur selektiven Hydrierung nach einem der Ansprüche 12 bis 14, derart, dass es bei einer Temperatur im Bereich von 20°C bis 200°C, unter einem Druck im Bereich von 0,4 MPa bis 5 MPa, mit einer stundenbezogenen Raumgeschwindigkeit (definiert als Verhältnis zwischen dem Volumendurchsatz der Charge und dem Katalysatorvolumen) im Bereich von 0,2 h⁻¹ und 30 h⁻¹ durchgeführt wird.
